(19) Europäisches Patentamt
European Patent Office
Office européen des brevets

(11) Veröffentlichungsnummer: **0 051 077**
**A1**

(12) # EUROPÄISCHE PATENTANMELDUNG

(21) Anmeldenummer: **80106676.2**

(22) Anmeldetag: **31.10.80**

(51) Int. Cl.³: **C 07 C 59/90**
C 07 C 59/88, C 07 C 59/84
C 07 C 51/09, C 07 C 51/41
C 07 C 51/02, C 07 C 51/38
A 61 K 31/19
//C07C69/738, C07C69/65,
C07C143/68, C07C67/343,
C07C67/333, C07C67/317,
C07C67/08, C07C67/00, C07C33/46,
C07C29/36

(30) Priorität: **31.10.80 CH 9785/79**

(43) Veröffentlichungstag der Anmeldung:
**12.05.82 Patentblatt 82/19**

(84) Benannte Vertragsstaaten:
**BE IT**

(71) Anmelder: **Byk Gulden Lomberg Chemische Fabrik GmbH**
**Byk-Gulden-Strasse 2**
**D-7750 Konstanz(DE)**

(72) Erfinder: **Kraas, Ekkehard, Dr.**
**Küsterkoppel 15**
**D-2401 Krummesse(DE)**

(72) Erfinder: **Ludwig, Gerhard, Dr.**
**Meisenweg 6**
**D-7750 Konstanz 16(DE)**

(72) Erfinder: **Rapp, Erich, Dr.**
**Pfarrer-Braun-Strasse 6**
**D-7760 Radolfzell 17(DE)**

(72) Erfinder: **Wolf, Horst, Dr.**
**Brandesstrasse 29**
**D-7750 Konstanz(DE)**

(54) **Substituierte Oxocarbonsäuren, Verfahren zu ihrer Herstellung, ihre Verwendung und sie enthaltende Arzneimittel.**

(57) Substituierte Oxocarbonsäuren der Formel I

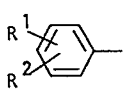 $-(CH_2)_n - CO - COOH$ (I),

worin $R^1$ ein Wasserstoffatom, ein Halogenatom, eine Hydroxygruppe, eine Niederalkylgruppe, eine Niederalkoxygruppe oder eine Trifluormethylgruppe, $R^2$ ein Wasserstoffatom oder ein Halogenatom und n eine ganze Zahl von 3 bis 8 bedeuten, und ihre Salze entfalten an Warmblütlern eine hypoglykämische Wirkung. Verfahren zur Herstellung der neuen Verbindungen und der zu ihrer Herstellung benötigten Zwischenprodukte sowie der entsprechenden Arzneimittel werden beschrieben.

EP 0 051 077 A1

Croydon Printing Company Ltd.

Substituierte Oxocarbonsäuren, Verfahren zu ihrer Herstellung, ihre Verwendung und sie enthaltende Arzneimittel

Die Erfindung betrifft substituierte Oxocarbonsäuren, Verfahren zu ihrer Herstellung , ihre Verwendung und sie enthaltende Arzneimittel.

In der deutschen Offenlegungsschrift DE-OS 2 365 755 werden α-Ketosäureester beschrieben, die als Zwischenprodukte für die Herstellung von Aminosäuren oder heterocyclischen Verbindungen dienen können. Aus der belgischen Patentschrift BE-PS 779 821 ist ein Verfahren zur Herstellung von α-Ketosäuren bekannt, wobei die Ketosäuren als Zwischenprodukte im Metabolismus oder als Vorstufen von Aminosäuren erwähnt werden. 5-(4-Methoxyphenyl)-2-oxovaleriansäure wurde von L.F. Fieser und H.L. Holmes [J.Amer.Chem.Soc. 58(1936)2319] hergestellt. Bestimmte substituierte Oxocarbonsäuren wurden nun als pharmazeutische Wirkstoffe mit einer spezifischen Wirkung erfunden.

Gegenstand der Erfindung sind substituierte Oxocarbonsäuren der allgemeinen Formel I

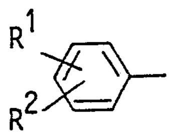 $(CH_2)_n$ - CO - COOH (I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom oder eine Methoxygruppe und $R^2$ ein Wasserstoffatom bedeuten, wenn n die Bedeutung 3 hat,

und ihre Salze.

Als Niederalkylgruppen kommen geradkettige oder verzweigte Alkylreste mit 1 bis 4 Kohlenstoffatomen in Betracht. Geradkettige Alkylreste sind beispielsweise der Methyl-, Ethyl-, n-Propyl- und n-Butylrest, von denen die mit 1 und 2 Kohlenstoffatomen bevorzugt sind. Verzweigte Alkylreste sind beispielsweise der Isopropyl-, Isobutyl- und sek.-Butylrest, von denen der

mit 3 Kohlenstoffatomen bevorzugt ist. Als Alkylreste von Niederalkoxygruppen kommen sowohl geradkettige als auch verzweigte Niederalkylgruppen in Frage. Die Methoxygruppe ist als Niederalkoxygruppe bevorzugt.

Halogenatome sind Fluor-, Chlor- und Bromatome, von denen Fluor, insbesondere Chlor, bevorzugt sind.

Die Substituenten $R^1$ und $R^2$ stehen bevorzugt in meta- oder para-Stellung zum Ketocarbonsäurerest.

Als Salze kommen Salze mit anorganischen und organischen Basen in Betracht. Pharmakologisch nicht verträgliche Salze werden nach an sich bekannten Methoden in pharmakologisch, d.h. biologisch, verträgliche Salze übergeführt, die unter den erfindungsgemäßen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminosäuren, etc. zu Anwendung.

Beispielsweise seien die Salze von Ethylendiamin, Dimethylamin, Diethylamin, Morpholin, Piperidin, Piperazin, N-Niedrigalkylpiperazin (z.B. N-Methylpiperazin), Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin und Chinolin, vorzugsweise von Lithium, Natrium, Kalium, Magnesium, Calcium und Aluminium, genannt.

Eine Ausgestaltung der Erfindung sind substituierte Oxocarbonsäuren der allgemeinen Formel I*

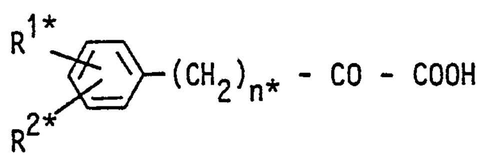

(I*),

worin

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom und

n* eine ganze Zahl von 3 bis 6 bedeuten, wobei nicht $R^{1*}$ ein Wasserstoffatom oder eine Methoxygruppe und $R^{2*}$ ein Wasserstoffatom bedeuten, wenn n* die Bedeutung 3 hat,

und ihre Salze.

Eine bevorzugte Ausgestaltung der Erfindung sind substituierte Oxocarbonsäuren der allgemeinen Formel I**

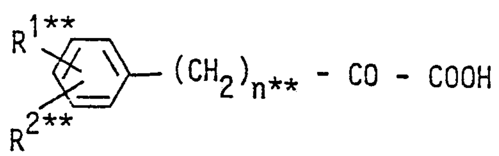 (I**),

worin $R^{1**}$ und $R^{2**}$ in meta- oder para-Stellung zum Ketocarbonsäurerest stehen und $R^{1**}$ ein Wasserstoffatom oder ein Chloratom, $R^{2**}$ ein Chloratom und n** 3 bis 5 bedeuten, und ihre pharmakologisch verträglichen Salze mit anorganischen oder organischen Basen.

Als Vertreter der erfindungsgemäßen Säuren seien beispielsweise 6-(2,4-Dichlorphenyl)-2-oxocapronsäure, 2-Oxo-6-(3-trifluormethylphenyl)-capronsäure, 5-(3,4-Dichlorphenyl)-2-oxovaleriansäure, 7-(3-Chlorphenyl)-2-oxoheptansäure, 7-(4-Methoxyphenyl)-2-oxoheptansäure, 7-(4-Bromphenyl)-2-oxoheptansäure, 8-(4-n-Butoxyphenyl)-2-oxo-octansäure, 8-(3-Fluorphenyl)-2-oxo-octansäure, 2-Oxo-8-(3-trifluormethylphenyl)-octansäure, 7-(4-Methylphenyl)-2-oxo-heptansäure, 8-(4-Hydroxyphenyl)-2-oxo-octansäure, 9-(4-Chlorphenyl)-2-oxo-nonansäure, 9-(3-Trifluormethylphenyl)-2-oxo-nonansäure, 10-(2,4-Dichlorphenyl)-2-oxo-decansäure, 10-(4-Methylphenyl)-2-oxo-decansäure genannt.

Bevorzugte Vertreter sind
2-Oxo-6-phenylcapronsäure,
6-(4-Methoxyphenyl)-2-oxo-capronsäure,
5-(4-Chlorphenyl)-2-oxo-valeriansäure
und ihre pharmakologisch verträglichen Salze.

Die erfindungsgemäßen Verbindungen weisen wertvolle pharmakologische Eigenschaften auf, die sie gewerblich verwertbar machen. Sie wirken hypoglycämisch.

Aufgrund ihrer vorteilhaften Wirksamkeit sind die erfindungsgemäßen substituierten Oxocarbonsäuren der allgemeinen Formel I bzw. I' sowie der Ausgestaltungen I* und I** sowie ihre Salze zur Behandlung und Prophylaxe von Krankheiten, die auf Störungen des Glucosestoffwechsels beruhen, geeignet. Beispielsweise werden behandelt prädiabetische Zustände zur Verhinderung der Manifestation des Diabetes, manifester Diabetes, z.B. Erwachsenendiabetes, labiler Diabetes von Jugendlichen.

Gegenstand der Erfindung ist daher auch eine Methode zur Bekämpfung der angegebenen Krankheiten durch Applikation der erfindungsgemäßen Verbindungen. Gegenstand der Erfindung ist ferner die Verwendung der erfindungsgemäßen Verbindungen bei der Bekämpfung der angegebenen Krankheiten.

Ein weiterer Gegenstand der Erfindung sind Arzneimittel, die eine oder mehrere der substituierten Oxocarbonsäuren der allgemeinen Formel I'

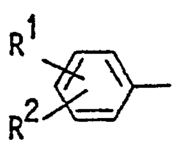 $(CH_2)_n$ - CO - COOH (I'),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten,

und/oder ihre pharmakologisch verträglichen Salze mit anorganischen oder organischen Basen enthalten.

Ausgestaltungen der Arzneimittel sind solche, die substituierte Oxocarbonsäuren der Formel I', in der $R^1$ ein Wasserstoffatom, ein Chloratom, eine Methylgruppe, eine Methoxygruppe oder eine Trifluormethylgruppe, $R^2$ ein Wasserstoffatom oder ein Chloratom und n eine ganze Zahl von 3 bis 6 bedeuten, I* oder I** oder die pharmakologisch verträglichen Salze der Säuren mit anorganischen oder organischen Basen, enthalten.

Gegenstand der Erfindung ist außerdem die Verwendung der erfindungsgemässen Verbindungen zur Herstellung von Arzneimitteln zur Bekämpfung der angegebenen Krankheiten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 1 bis 95, vorzugsweise 15 bis 85 Gewichtsprozent der Gesamtmischung.

In Übereinstimmung mit der Erfindung können im humanmedizinischen Bereich die Wirkstoffe in beliebiger Form, z.B. systemisch, angewandt werden unter der Voraussetzung, daß die Ausbildung bzw. Aufrechterhaltung von ausreichenden Blut- oder Gewebsspiegeln an Wirkstoffen gewährleistet ist. Das kann beispielsweise durch orale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachem einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung enthalten, wenn sie in Einheitsdosen vorliegen und für die Applikation, z.B. am Menschen bestimmt sind, etwa 2 bis 200 mg, vorteilhafterweise 10 bis 100 mg und insbesondere 20 bis 60 mg Wirkstoff.

Im allgemeinen hat es sich in der Humanmedizin als vorteilhaft erwiesen, den oder die Wirkstoffe bei oraler Gabe in einer Tagesdosis von etwa 0,1 bis etwa 30, vorzugsweise 0,3 bis 15, insbesondere 0,6 bis 3 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von etwa 0,05 bis etwa 10, vorzugsweise 0,1 bis 5, insbesondere 0,3 bis 1 mg/kg Körpergewicht.

Bei einer parenteralen Behandlung, z.B. einer intravenösen oder intramusculären Applikation können ähnliche Dosierungen zur Anwendung kommen. Bei dieser Therapie werden etwa 0,3 bis 1 mg Wirkstoff/kg Körpergewicht appliziert.

Die therapeutische Verabreichung der pharmazeutischen Zubereitung erfolgt bei Dauermedikation im allgemeinen zu festgelegten Zeitpunkten, wie 1 bis 4 mal am Tage, z.B. jeweils nach den Mahlzeiten und/oder am Abend. Bei akuten Anlässen erfolgt die Medikation zu variierendem Zeitpunkt. Unter

bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikation der Wirkstoffe nimmt der Fachmann aufgrund seines Fachwissens vor.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wäßrige und ölige Suspensionen, Emulsionen oder Lösungen kommen.

Tabletten können inerte Verdünnungsmittel, z.B. Calciumcarbonat, Calciumphosphat, Natriumphosphat oder Xylit; Granulierungs- und Verteilungsmittel, z.B. Calciumphosphat oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß eine verzögerte Auflösung und Resorption des Arzneimittels im Gastrointestinaltrakt und damit z.B. eine bessere Verträglichkeit, Protrahierung oder eine Retardierung erreicht wird. Gelatinekapseln können den Arzneistoff vermischt mit einem festen Verdünnungsmittel, z.B. Calciumcarbonat oder Kaolin, oder einem öligen Verdünnungsmittel, z.B. Paraffinöl, enthalten.

Wäßrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharin, Natriumcyclamat, enthalten.

Ölige Suspensionen können z.B. Paraffinöl und Verdickungsmittel, wie Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den obengenannten, sowie mit Süßungsmitteln, Geschmacksmitteln und Farbstoffen enthalten.

Emulsionen können z.B. Paraffinöl neben Emulgiermitteln, wie Akaziengummi, Traganthgummi, Phosphatiden, Sorbitanmonooleat, Polyoxyethylensorbitanmonooleat, und Süßungs- und Geschmacksmitteln enthalten.

Zur parenteralen Anwendung der Arzneistoffe dienen steril injizierbare wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglycol, enthalten können.

Der oder die Wirkstoffe können gegebenenfalls mit einem oder mehreren der angegebenen Träger- oder Zusatzstoffe auch in mikroverkapselter Form formuliert werden.

Neben den erfindungsgemäßen substituierten Oxocarbonsäuren, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe u.a.), z.B. Carbutamid, Tolbutamid, Chlorpropamid, Glibenclamid, Glibornurid, Glisoxepid, Gliquidon, Glymidin oder Hypolipidämika, wie Nikotinsäure sowie deren Derivate und Salze enthalten.

Ein weiterer Gegenstand der Erfindung ist ein Verfahren zur Herstellung der substituierten Oxocarbonsäuren der allgemeinen Formel I

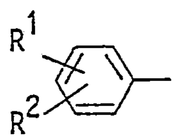 $(CH_2)_n$ - CO - COOH (I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom oder eine Methoxygruppe und $R^2$ ein Wasserstoffatom bedeuten, wenn n die Bedeutung 3 hat,

und ihrer Salze, dadurch gekennzeichnet, daß man

a) einen Oxocarbonsäureester der allgemeinen Formel II

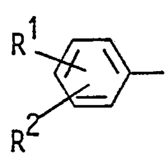 $(CH_2)_n$ - CO - CO - O - $R^3$ (II),

worin $R^1$, $R^2$ und n die vorstehend angegebene Bedeutung haben und $R^3$ einen Niederalkylrest bedeutet, solvolysiert und gegebenenfalls anschließend erhaltene Säuren in die Salze oder erhaltene Salze in die Säuren überführt, oder

b) einen Diester der allgemeinen Formel III

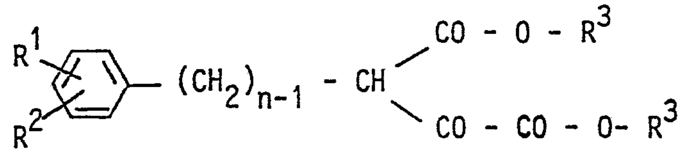 (III),

worin $R^1$, $R^2$, $R^3$ und n die vorstehend angegebene Bedeutung haben, solvolysiert und decarboxyliert und gegebenenfalls anschließend erhaltene Säuren in die Salze oder erhaltene Salze in die Säuren überführt.

Die Verbindungen der allgemeinen Formel I werden nach an sich bekannten Verfahren hergestellt. Die Herstellung der Verbindungen I kann neben den angegebenen Verfahrensvarianten auch analog anderen in der Literatur beschriebenen Verfahren erfolgen, beispielsweise seien genannt: Die in der BE-PS 779 821 angegebenen Methoden, die Verfahren von H. Poisel [Ber. 111 (1978)3136], R. Fischer und T. Wieland [Ber. 93 (1960) 1387], J. Anatol und A. Medête [Synthesis 1971, 538], E.E. Eliel und A.A. Hartmann [J.org. Chem. 37 (1972) 505], K. Tanaka et al. [Tetrahedron Letters 1978, 4809], K. Ogura et. al. [Tetrahedron Letters 1978, 375].

Die Solvolyse der Oxocarbonsäureester II und der Diester III erfolgt beispielsweise mit einer wässerigen oder alkoholischen (z.B. ethanolischen) Alkalimetallhydroxid- (z.B. Kaliumhydroxid-)Lösung bei Raumtemperatur, gegebenenfalls unter Zusatz eines inerten Verdünnungsmittels, wie Dioxan oder Toluol. Bevorzugt erfolgt die Solvolyse mit wässerigen Lösungen von Mineralsäuren, wie Salzsäure, Bromwasserstoffsäure, Schwefelsäure, gegebenenfalls unter Zusatz eines organischen Lösungsmittels, wie Dioxan oder Diglyme, bei Temperaturen zwischen 0°C und der Siedetemperatur des Lösungsmittels, bevorzugt zwischen 20 und 70°C. Die Decarboxylierung der Diester III erfolgt durch Erhitzen der jeweiligen Lösungen, gegebenenfalls gleichzeitig mit der Solvolyse.

Die Überführung der Oxocarbonsäuren der allgemeinen Formel I bzw. der Ausgestaltungen I* und I** in die Salze kann durch direkte alkalische Solvolyse, z.B. Hydrolyse, der Ester II ($R^3$ = Niederalkyl) erfolgen. Als alkalischer Reaktionspartner wird diejenige anorganische oder organische Base verwendet, deren Salz gewünscht wird. Man erhält die Salze aber auch, indem man die Säuren I mit dem stöchiometrischen Äquivalent an entsprechender Base, z.B. Natriumhydroxid oder Natriumethanolat umsetzt, oder leicht lösliche Salze durch doppelte Umsetzung in schwer lösliche Salze überführt, oder beliebige Salze in pharmakologisch verträgliche Salze überführt. Die Herstellung der freien Oxosäuren I ist gegenüber der der Salze bevorzugt.

Zur Herstellung der substituierten Oxocarbonsäuren der Ausgestaltungen I* und I** werden Oxocarbonsäureester der allgemeinen Formeln II* und II** bzw. Diester III* und III**

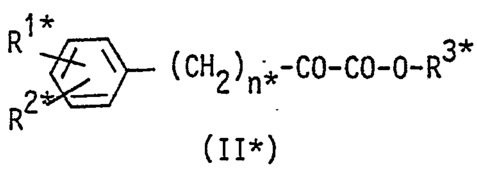

(II*)

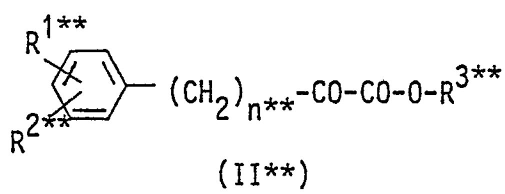

(II**)

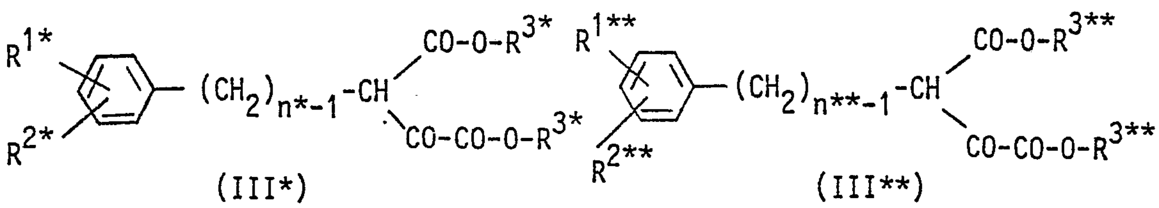

(III*) (III**)

worin $R^{1*}$, $R^{2*}$ und n* bzw. $R^{1**}$, $R^{2**}$ und n** die oben angegebene Bedeutung haben, $R^{3*}$ einen Niederalkylrest und $R^{3**}$ einen Methyl- oder Ethylrest bedeutet, eingesetzt.

Die Herstellung der Oxocarbonsäureester der allgemeinen Formeln II, II* und II** erfolgt nach an sich bekannten Verfahren, z.B. gemäß der DE-OS 23 65 755. Sie kann auch durch Ozonolyse von α-Methylencarbonsäureestern der allgemeinen Formel IV

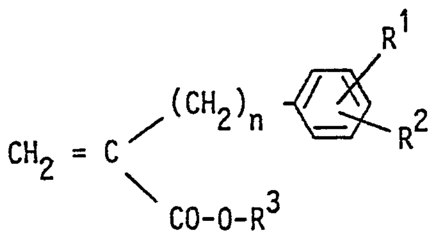

(IV),

worin $R^1$, $R^2$, $R^3$ und n die oben angegebene Bedeutung hat, erfolgen. Die α-Methylencarbonsäureester IV (bzw. die entsprechenden Ausgestaltungen IV* und IV**) werden in Analogie zu den von H. Stetter und H. Kuhlmann [Synthesis 1978, 29], Ph.E. Pfeffer et al. [J.Org.Chem. 37 (1972) 1256] und W.S. Wadsworth, jun. und W.D. Emmons [J.Amer.Chem.Soc. 83 (1961) 1733] beschriebenen Methoden hergestellt.

Die Herstellung der Diester III erfolgt nach Methoden wie sie dem Fachmann geläufig sind, beispielsweise durch Umsetzung von Carbonsäureestern V mit Dialkyloxalat VI

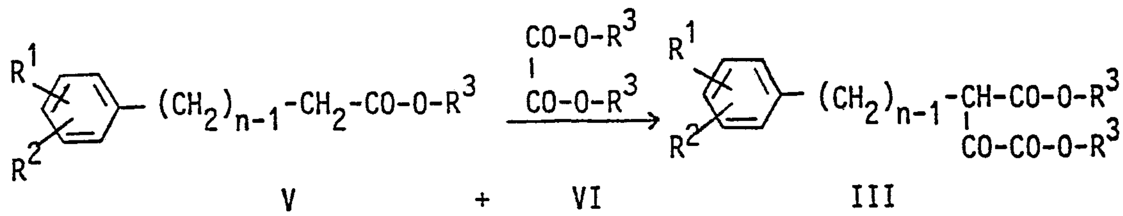

V + VI III

wobei $R^1$, $R^2$, $R^3$ und n die oben angegebene Bedeutung haben, in Gegenwart einer starken Base, z.B. Natriumethanolat oder Kalium-tert.-butylat. Die Carbonsäureester V sind nach bekannten Methoden, z.B. nach R. Huisgen et al. Liebigs Annalen 586(1954)52, zugänglich.

Die folgenden Beispiele dienen zur Erläuterung der Erfindung, ohne sie zu beschränken. Kp. bedeutet Siedepunkt, F. bedeutet Schmelzpunkt. Temperaturangaben erfolgen in °C.

## Beispiele

### Beispiel 1

2-Oxo-6-phenylcapronsäure

a) 10,0 g des nach b) erhaltenen Diesters werden zusammen mit 100 ml 6 N Salzsäure und 100 ml Dioxan 4 Stunden auf ca. 100°C erhitzt. Nach Abkühlen verdünnt man mit 1,2 l Wasser und extrahiert mehrmals mit Methylenchlorid. Die organische Phase wird mit Wasser und gesättigter Kochsalzlösung gewaschen, dann getrocknet und eingeengt. Der ölige Rückstand wird durch Hochvakuumdestillation [Kp. 130-135° bei 0,1 Torr (13,3 Pa)] und Säulenchromatographie (Laufmittel: Chloroform) gereinigt. Es verbleiben 2,9 g 2-Oxo-6-phenylcapronsäure als nicht kristallisierendes Öl, Ausbeute 43 % (d.Th.).

b) Zu einer Natriumethylatsuspension, hergestellt aus 6,4 g Natrium und 100 ml Ethanol mit anschließendem Abdampfen überschüssigen Ethanols und Zugabe von 100 ml Toluol, gibt man 47,9 g 5-Phenylvaleriansäureethylester. Unter Rühren tropft man 44,1 g Diethyloxalat zu und erhitzt 2,5 Stunden auf Rückfluß. Nach Abkühlen wird die Lösung mit 500 ml Eiswasser verrührt und mit 2 N Schwefelsäure auf pH 2 angesäuert. Mehrfache Extraktion mit Methylenchlorid ergibt nach Trocknen, Filtration durch eine kurze Kieselgel-Säule und Einengen 62,1 g 3-Ethoxycarbonyl-2-oxo-6-phenylhexansäureethylester, der ohne weitere Reinigung gemäß a) umgesetzt wird.

### Beispiel 2

6-(4-Methoxyphenyl)-2-oxocapronsäure

a) Analog Beispiel 1a) hydrolysiert und decarboxyliert man den nach b) erhaltenen Diester mit 6 N Salzsäure. Die gesammelten Methylenchloridextrakte engt man ein, löst den Rückstand in Diethylether und extrahiert mehrmals mit 1 N Natronlauge. Die wäßrigen Phasen werden mit 6 N Salzsäure auf pH 5,5 angesäuert und dreimal mit Diethylether gewaschen. Darauf bringt man die wäßrige Phase auf pH 1-2 und extrahiert mit Methylenchlorid. Nach Trocknen und Einengen verbleibt ein zähes Öl, das aus Petrolether (50-70°C) in der Kälte kristallisiert. Ausbeute 6,2 g (51 % d.Th.), F. 22-25°.

b) Analog Beispiel 1b) werden 17,2 g 3-Ethoxycarbonyl-6-(4-methoxyphenyl)-2-oxocapronsäureethylester aus 14,7 g 5-(4-Methoxyphenyl)-valeriansäureethylester und 11,8 g Diethyloxalat mit Natriumethylat als Base hergestellt.

Beispiel 3

5-(4-Chlorphenyl)-2-oxovaleriansäure

a) 5-(4-Chlorphenyl)-2-oxovaleriansäureethylester (Rückstand aus b) wird in 100 ml 6 N Salzsäure und 100 ml Dioxan aufgenommen und 3 Stunden auf 100$^{o}$ erhitzt. Nach Abkühlen verdünnt man mit 1 l Wasser und extrahiert mehrmals mit Methylenchlorid. Die organische Phase wird mehrmals mit Wasser gewaschen, über Magnesiumsulfat getrocknet und eingeengt. Der Rückstand wird im Kugelrohrofen hochvakuumdestilliert und aus Petrolether umkristallisiert. Man erhält 5,7 g der Titelverbindung vom F. 82-84$^{o}$.

b) 14 g 5-(4-Chlorphenyl)-2-methylenvaleriansäureethylester werden in 350 ml Ethanol gelöst und mit der äquivalenten Menge Ozon behandelt. Nach Spülen der Apparatur mit Stickstoff gibt man ca. 1 g Palladium auf Kohle (10 % Pd) zu und reduziert das entstandene Ozonid im Umlaufverfahren mit Wasserstoff. Anschließend filtriert man den Katalysator ab und engt die Lösung im Vakuum ein.

c) Analog der von H. Stetter und H. Kuhlmann [Synthesis 1979, 29] beschriebenen Methode erhält man aus 71 g 3-(4-Chlorphenyl)-propylmalonsäuremonoethylester, 10,42 g Paraformaldehyd, 47 ml Pyridin und 3,1 ml Piperidin 53,3 g 5-(4-Chlorphenyl)-2-methylenvaleriansäureethylester vom Kp. 120-123$^{o}$ bei 0,05 Torr (6,65 Pa).

d) Man tropft bei Raumtemperatur eine Lösung von 16,8 g Kaliumhydroxid in 400 ml Ethanol zu 92 g 3-(4-Chlorphenyl)-propylmalonsäurediethylester in 200 ml Ethanol. Man rührt 24 Stunden, engt im Vakuum weitgehend ein, nimmt den Rückstand in 500 ml Wasser auf und extrahiert 2 mal mit je 100 ml Diethylether. Die wässerige Phase wird unter Eiskühlung mit konzentrierter Salzsäure angesäuert und 3mal mit je 200 ml Diethylether extrahiert; die organische Phase wird nach dem Trocknen

über Natriumsulfat eingeengt. Zurück bleiben 71,8 g 3-(4-Chlorphenyl)-propylmalonsäureethylester als viskoses Öl.

e) Man tropft bei 50° 108,5 g Malonsäurediethylester zu einer aus 15,6 g Natrium und 750 ml Ethanol frisch hergestellten Natriumethylatlösung. Man hält 2,5 Stunden bei dieser Temperatur und tropft anschließend 220 g p-Toluolsulfonsäure-[3-(4-chlorphenyl)-propyl]-ester zu. Nach beendeter Zugabe rührt man 6 Stunden bei 50°, versetzt anschließend mit 800 ml Wasser und extrahiert 3 mal mit insgesamt 1 Liter Diethylether. Die vereinigten organischen Phasen werden nach dem Trocknen über Natriumsulfat und Abdampfen des Lösungsmittels destilliert. Man erhält 105,6 g 3-(4-Chlorphenyl)-propylmalonsäurediethylester vom Kp. 145-155° bei 0,01 Torr (1,33 Pa).

f) Man tropft bei 0° 135 ml Pyridin zu 150 g 3-(4-Chlorphenyl)-propanol-1 und 206,6 g p-Toluolsulfonsäurechlorid in 300 ml Chloroform. Nach beendeter Zugabe rührt man 3 Stunden bei Raumtemperatur und gießt die Lösung in eine Mischung aus 400 ml Wasser und 120 ml konzentrierter Salzsäure. Die organische Phase wird abgetrennt, 3 mal mit Wasser gewaschen, über Natriumsulfat getrocknet und im Vakuum zu einem gelblichen viskosen Öl eingeengt, Ausbeute 285 g p-Toluolsulfonsäure-[3-(4-chlorphenyl)-propyl]-ester.

Beispiel 4

2-Oxo-7-(3-trifluormethylphenyl)-heptansäure

a) Analog Beispiel 1a) hydrolysiert und decarboxyliert man den nach b) erhaltenen Diester mit 6 N Salzsäure in Dioxan bei 100°. Nach extraktiver Aufarbeitung und Reinigung durch Kugelrohrdestillation erhält man 5,6 g der Titelverbindung als zähes, farbloses Öl, das nicht kristallisiert.

b) Analog Beispiel 1b) wird 3-Ethoxycarbonyl-2-oxo-7-(3-trifluormethylphenyl)-heptansäureethylester aus 10,9 g 6-(3-Trifluormethylphenyl)-capronsäureethylester und 6 g Diethyloxalat mit Natriumethylat als Base erhalten, Ausbeute 11,7 g (80 % d.Th.).

c) 11,4 g 6-(3-Trifluormethylphenyl)-capronsäure werden in 200 ml Ethanol gelöst und nach Zugabe von 2 ml konzentrierter Schwefelsäure 6 Stunden auf Rückfluß erhitzt. Nach Abdestillation der Hauptmenge des Lösungsmittels wird mit Wasser versetzt und mit Methylenchlorid extrahiert. Behandeln mit Natriumhydrogencarbonatlösung, Trocknen und Einengen ergibt 12,6 g des entsprechenden Ethylesters als Öl, der ohne weitere Reinigung in b) eingesetzt wird.

d) 24,9 g 4-(3-Trifluormethylphenyl)-butylmalonsäurediethylester werden mit 4,5 g Kaliumhydroxid in 200 ml Toluol:Methanol (2:1) 36 Stunden am Rückfluß erhitzt und anschließend sauer extrahiert. Der nach Trocknen und Einengen verbleibende Rückstand wird im Vakuum 2 Stunden auf ca. $170^0$ erhitzt; dabei wird Kohlendioxyd frei und es bildet sich die entsprechende Capronsäure, die in c) eingesetzt wird.

e) In eine aus 28,5 g 2-(3-Trifluormethylphenyl)-ethylbromid und 2,9 g Magnesium hergestellte Grignard-Lösung in 200 ml Diethylether tropft man 5,5 g Oxiran in 20 ml Diethylether bei ca. $0^oC$. Nach 1 Stunde Rühren und Versetzen mit 100 ml 10 %iger Schwefelsäure extrahiert man mit Diethylether und destilliert den Rückstand. Man erhält ca. 18,5 g 4-(3-Trifluormethylphenyl)-butan-1-ol, das in 70 ml Toluol gelöst und mit 16 g p-Toluolsulfonsäurechlorid und 25 ml Pyridin versetzt und gerührt wird. Nach 2 Tagen wird vom Niederschlag abfiltriert und extraktiv aufgearbeitet. Nach Einengen der organischen Phase bleiben 25,7 g Tosylat des substituierten Butanols zurück, das in 250 ml Ethanol aufgenommen und zu einer aus 11,5 g Diethylmalonat und 1,6 g Natrium in 220 ml absolutem Ethanol hergestellten Lösung zugetropft wird. Die Mischung wird 24 Stunden auf Rückfluß erhitzt, danach eingeengt und zwischen Wasser und Methylenchlorid verteilt. Nach mehrmaliger Extraktion werden die gesammelten organischen Phasen getrocknet und eingeengt. Der Rückstand wird über eine 500 g Kieselgelsäule chromatografisch gereinigt und ergibt 24,9 g 4-(3-Trifluormethylphenyl)-butylmalonsäurediethylester, der in d) eingesetzt wird.

Beispiel 5

9-(2,4-Dimethylphenyl)-2-oxo-nonansäure

a) 8,6 g des nach b) erhaltenen Diesters werden nach der in Beispiel 1a) genannten Methode mit 6 N Salzsäure und Dioxan hydrolysiert und decarboxyliert. Extraktion mit Methylenchlorid, gefolgt von Waschen, Trocknen und Einengen, liefert nach Reinigung durch Hochvakuumdestillation 4,9 g der Titelverbindung als zähes Öl.

b) 7,5 g des nach c) erhaltenen Octansäureesters werden analog Beispiel 1b) mit Natriumethylat und Diethyloxalat umgesetzt. Nach Ansäuern, Extrahieren mit Methylenchlorid, Trocknen und Filtrieren durch Kieselgel verbleiben nach Entfernen des Lösungsmittels 8,6 g 9-(2,4-Dimethylphenyl)-3-ethoxycarbonyl-2-oxononansäureethylester.

c) 12,8 g 8-(2,4-Dimethylphenyl)-7-oxooctansäureethylester [erhalten nach d)] werden analog der Methode von Huisgen [R. Huisgen et al., Liebigs Ann. 586 (1954)52] mit Hydrazinhydrat in Diethylenglykol reduziert. Das isolierte Reduktionsprodukt wird in 200 ml Ethanol und 5 ml konzentrierter Schwefelsäure über Nacht zum Rückfluß erhitzt und liefert nach üblicher Aufarbeitung 7,5 g 8-(2,4-Dimethylphenyl)-octansäureethylester als farbloses Öl.

d) Aus 14,5 g 2,4-Dimethylbenzylbromid wird nach der Methode von Huisgen mit Magnesium und Cadmiumchlorid das entsprechende Cadmium-dialkyl hergestellt. Anschließend setzt man dieses in benzolischer Lösung mit 11,2 g Pimelinsäureethylesterchlorid in der Siedehitze um. Nach Versetzen mit Schwefelsäure wird die organische Phase gewaschen und das Benzol abgedampft. Der Rückstand wird im Hochvakuum destilliert und liefert 12,8 g 8-(2,4-Dimethylphenyl)-7-oxooctansäureethylester.

Beispiel 6

10-(4-Chlorphenyl)-2-oxodecansäure

a) 5,7 g des nach b) erhaltenen Diesters werden nach Beispiel 1a) mit 6 N Salzsäure in Dioxan hydrolysiert und decarboxyliert. Nach Aufarbeitung und Reinigung durch Säulenchromatographie verbleiben 3,8 g der Titelverbindung als farbloses Öl.

b) 5,3 g der nach c) erhaltenen Verbindung werden nach Beispiel 1b) mit Diethyloxalat und Natriumethylat umgesetzt. Aufarbeitung und Reinigung durch Kieselgel-Filtration liefern nach Eindampfen des Lösungsmittels 5,7 g 10-(2,4-Dimethylphenyl)-3-ethoxycarbonyl-2-oxodecansäureethylester.

c) 8,3 g 9-(4-Chlorphenyl)-9-oxo-nonansäureethylester [erhalten nach d)] werden analog Beispiel 5c) mit Hydrazinhydrat reduziert und aufgearbeitet. Man erhält 5,3 g 9-(4-Chlorphenyl)-nonansäureethylester als Öl.

d) Nach der von Papa et al. [J.Amer.Chem.Soc. 69 (1947)3018] beschriebenen Methode werden 12,3 g Azelainsäureethylesterchlorid in 20 ml Chlorbenzol mit 9,5 g Aluminiumtrichlorid in der Kälte zusammengegeben und dann über Nacht auf $150^0$ erhitzt. Nach Versetzen des Komplexes mit verdünnter Salzsäure wird die organische Phase gewaschen und eingeengt; der Rückstand wird mit Diethylether extrahiert. Nach Säulenchromatographie und Einengen der Lösung werden 8,3 g 9-(4-Chlorphenyl)-9-oxo-nonansäureethylester als zähes Öl erhalten.

Beispiel 7

6-(4-Methoxyphenyl)-2-oxocapronsäure-Natriumsalz

4,5 g 6-(4-Methoxyphenyl)-2-oxocapronsäure werden in 19,0 ml 1 N Natronlauge 15 Minuten bei Raumtemperatur aufgerührt. Die Mischung wird filtriert, einmal mit Diethylether gewaschen und zur Trockne eingedampft. Der bei $30^0$ im Vakuum getrocknete Rückstand besteht aus dem reinen Natriumsalz.

Beispiel 8

5-(4-Chlorphenyl)-2-oxovaleriansäure-calciumsalz

5,3 g 5-(4-Chlorphenyl)-2-oxovaleriansäure werden in 25 ml 1 N Natronlauge gelöst und mit wenig halbkonzentrierter Salzsäure auf pH 8,5 eingestellt. Durch Zugabe von 2,0 g Calciumchlorid-dihydrat in 6 ml Wasser unter Rühren wird das Calciumsalz gefällt, das nach Filtrieren im Vakuum bei $40^0$ getrocknet wird.

Beispiel 9

10 000 Kapseln mit einem Wirkstoffgehalt von 25 mg werden wie folgt hergestellt:

250 g 5-(4-Chlorphenyl)-2-oxovaleriansäure werden in 3000 ml Methylenchlorid gelöst. Die Lösung wird mit 750 g mikronisierter Kieselsäure gut gemischt. Die Mischung wird zur Trockne eingedampft und dann in Hartgelatinekapseln Größe 4 abgefüllt.

Beispiel 10

10 000 Tabletten mit einem Wirkstoffgehalt von 30 mg werden wie folgt hergestellt:

300 g 5-(4-Chlorphenyl)-2-oxovaleriansäure, 800 g Xylit, 500 g Calciumphosphat, 30 mg amorphe Kieselsäure und 40 g Natriumlaurylsulfat werden gemischt und gesiebt. Diese Mischung wird mit einer Lösung von 50 g Polyvinylpyrrolidon (mittleres Mol.-Gewicht 25 000) in 320 ml Ethanol befeuchtet und durch ein Sieb mit der Maschenweite 1,25 mm granuliert. Das Granulat wird bei $40^0$ getrocknet und mit 160 g Pektin, 100 g Talk und 20 g Magnesiumstearat gemischt. Diese Mischung wird zu Tabletten à 200 mg mit 8 mm Durchmesser verpreßt.

Beispiel 11

10 000 Kapseln mit einem Wirkstoffgehalt von 25 mg werden wie folgt hergestellt:

250 g 5-(4-Methoxyphenyl)-2-oxovaleriansäure, 495 g mikrokristalline Cellulose und 255 g amorphe Kieselsäure werden gut gemischt und in Hartgelatinekapseln Größe 4 abgefüllt.

## Pharmakologie

Die erfindungsgemäßen Verbindungen senken infolge ihrer insulinotropen Wirkung den Blutglukosespiegel, wobei sie sich in ihrer chemischen Struktur grundlegend von pankreaswirksamen, betacytotropen Substanzen (z.B. Sulfonylharnstoffen) unterscheiden. Als besonders vorteilhaft erweist sich, daß der insulinotrope Effekt der Verbindungen im Gegensatz zu handelsüblichen Sulfonylharnstoffen von der Glukosekonzentration des umgebenden Mediums abhängig ist. Die erfindungsgemäßen Verbindungen zeigen somit die Voraussetzungen für ein sogenanntes "denkendes Antidiabetikum", das die Insulinabgabe nur bei Hyperglykämie stimuliert, während es bei euglykämischen Bedingungen keine zusätzliche Insulinabgabe hervorruft. Unter solchen Voraussetzungen wird die Gefahr einer Hypoglykämie ausgeschlossen.

In der anschließenden Tabelle werden die untersuchten Verbindungen durch eine laufende Nummer bezeichnet, die wie folgt zuzuordnen ist:

| Lfd. Nr. | Name der Verbindung |
|---|---|
| 1 | Tolbutamid [$N^1$-n-Butyl-$N^2$-(4-methylphenylsulfonyl)-harnstoff] |
| 2 | 5-(4-Methoxyphenyl)-2-oxo-valeriansäure |
| 3 | 6-(4-Methoxyphenyl)-2-oxo-capronsäure |
| 4 | 5-(4-Chlorphenyl)-2-oxovaleriansäure |

In der Tabelle I werden Untersuchungen der Insulinsekretion aus dem isoliert perfundierten Rattenpankreas innerhalb 60 Minuten bei alleiniger Zugabe von Glukose und bei Zusatz von Vertretern der erfindungsgemäßen Verbindungen zum Perfusat wiedergegeben.

Tabelle I

Insulinausschüttung aus dem isoliert perfundierten Rattenpankreas

| Zusätze zum Perfusat | | Insulinabgabe I | Verstärkungseffekt |
|---|---|---|---|
| Verb.-Nr. (in [mmol/Liter]) | Glukose [mmol/Liter] | [ng/60 Min.] | $I^+/I^-$ |
| - | 4 | 23 | - |
| - | 8,3 | 55 | - |
| 1(1) | 4 | 240 | 10,4 |
| 1(1) | 8,3 | 470 | 8,5 |
| 2(0,42) | 4 | 23 | 1 |
| 2(0,42) | 8,3 | 260 | 4,7 |
| 3(0,42) | 8,3 | 270 | 4,9 |
| 4(0,42) | 8,3 | 430 | 7,8 |

Zu Tabelle I:

$I^+$ = Insulinabgabe bei Zusatz der Testverbindungen und Glukose

$I^-$ = Insulinabgabe ohne Zusatz der Testverbindungen, jedoch bei Zusatz von Glukose

Aus Tabelle I ist ersichtlich, daß die Insulinabgabe bei alleinigem Zusatz von Glukose bei einer Konzentration von 8,3 mmol/l gegenüber dem von 4 mmol/l um den Faktor 2,3 gesteigert ist. Gleichzeitiger Tolbutamid-Zusatz steigert die Insulinausschüttung bei diesen Glukosekonzentrationen um die Faktoren 10,4 bzw. 8,5. Die erfindungsgemäße Verbindung 2 dagegen ist bei einer Glukosekonzentration von 4 mmol/l ohne Einfluß auf die Insulinsekretion, während sie bei einer Glukosekonzentration von 8,3 mmol/l eine deutliche Steigerung der Insulinausschüttung (auf das 4,7 fache) bewirkt. Eine fast gleich starke bzw.

eine höhere Steigerungsrate der Insulinabgabe werden bei Zusatz der erfindungsgemäßen Verbindungen 3 bzw. 4 erzielt.

Die Ermittlung der pharmakologischen Eigenschaften erfolgte nach folgender Methode:

A. Versuchstiere

Als Versuchstiere dienen gefastete, männliche Sprague-Dawley-Ratten (220-280 g) vom Stamm mus rattus, die unter Standardbedingungen (Tag-Nachtrhytmus 12h/12h, $23^{o}C$, 55 % Luftfeuchtigkeit, Wasser und Standarddiät Altromin$^{R}$ ad libitum) gehalten werden. 18 bis 20 Stunden vor Versuchsbeginn wird den Ratten das Futter entzogen.

B. Perfusionsmedium

Die Perfusion des Rattenpankreas wird analog der von Lenzen beschriebenen Methode [Amer.J.Physiol. 236(1979)E391-E400] durchgeführt. Als Perfusionsmedium wird Krebs-Henseleit-Puffer verwendet, der 1 mg/ml Rinderserumalbumin (Serva) und Glukose, wie angegeben, enthält. Das Perfusionsmedium wird in Gaswaschflaschen mit Carbogen ($95\%O_2/5\%CO_2$) ständig bei $37,5^{o}C$ und pH 7,4 equilibriert.

C. Perfusion und Operation

Die Ratten werden mit Nembutal (0.8 ml/kg) anästhesiert. Das Pankreas wird mit dem Magen, der anliegenden Duodenalschleife sowie dem versorgenden und ableitenden Gefäßsystem chirurgisch isoliert. Die Kanülierung erfolgt retrograd durch die Aorta. Alle abzweigenden Gefäße werden so ligiert, daß die Perfusionslösung durch die arteria coeliaca den Pankreas erreicht, ihn durchströmt, in der vena lienalis und der vena pancreatico-duodenalis gesammelt wird und dann in die vena porta mündet. Die Poartalvene wird wiederum retrograd kanüliert und hier wird das Perfusat fraktioniert aufgefangen.
Das isolierte Organpräparat wird in ein thermostatisiertes Glasgefäß mit einer am Boden gelegenen Öffnung zur Aufnahme der Ablaufkapillare überführt. Das Pankreas wird mit einem konstanten Fluß von 4 ml/min. infundiert. Wenn mindestens 3,7 ml wieder aufgefangen werden, wird das Präparat zum Experiment verwendet. Nach einer Vorperfusion von 10 Minuten, in der das Pankreas blutfrei gespült wird, wird für die Dauer von 20 Minuten ohne Substanz bei angegebener Glucosekonzentration und für die Dauer von 60 Minuten mit konstanter Testsubstanzkonzentration und der angegebenen Glucosekonzentration perfundiert. Das Perfusat wird jeweils zunächst für die Dauer von 10 Minuten in 2 Minuten-Abständen und darauffolgend in 5 Minuten-Abständen gesammelt.

D. Insulinbestimmung

Die Insulinbestimmung wird mit dem Radioimmunoassay der Firma Becton und Dickinson durchgeführt. Dieser Test arbeitet mit Meerschweinchenantikörpern gegen Schweineinsulin und mit $^{125}I$-markiertem Schweineinsulin. Das freie Antigen wird in diesem Test durch Adsorption an Dextran-beschichtete Aktivkohle und nachfolgende Zentrifugation getrennt. Als Standard für die Eichkurve wird hochreines Ratteninsulin der Firma Novo verwendet.

Aus den Insulinkonzentrationen der einzelnen Perfusatfraktionen wird die innerhalb 60 Minuten ausgeschiedene Menge an Insulin errechnet. Bei Perfusion ohne Substanz erwiesen Vorversuche, daß die Insulinsekretion nach 10 Minuten konstant bleibt. Der Efflux zwischen Minute 11 bis 60 wird dann aus dem Efflux zwischen Minute 11 bis 20 extrapoliert. Das Präparat wird dadurch kürzere Zeit belastet, gleichzeitig erhält man für jeden Versuch die Kontrolle im gleichen Experiment.

## Patentansprüche

1. Substituierte Oxocarbonsäuren der allgemeinen Formel I

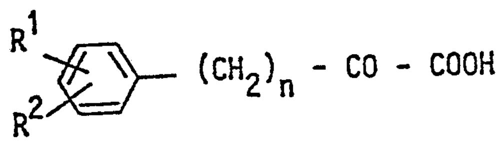 (I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom oder eine Methoxygruppe und $R^2$ ein Wasserstoffatom bedeuten, wenn n die Bedeutung 3 hat,

und ihre Salze.

2. Verbindungen nach Anspruch 1, worin n eine ganze Zahl von 3 bis 6 bedeutet, wobei nicht $R^1$ ein Wasserstoffatom oder eine Methoxygruppe und $R^2$ ein Wasserstoffatom bedeuten, wenn n die Bedeutung 3 hat.

3. Substituierte Oxocarbonsäuren nach Anspruch 1, gekennzeichnet durch die allgemeine Formel I*

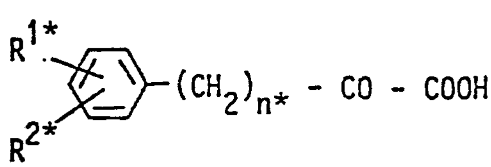 (I*)

worin

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom und

n* eine ganze Zahl von 3 bis 6 bedeuten, wobei nicht $R^{1*}$ ein Wasser-

stoffatom oder eine Methoxygruppe und $R^{2*}$ ein Wasserstoffatom bedeuten, wenn n* die Bedeutung 3 hat,
und ihre Salze.

4. Substituierte Oxocarbonsäuren nach Anspruch 1, gekennzeichnet durch die allgemeine Formel I**

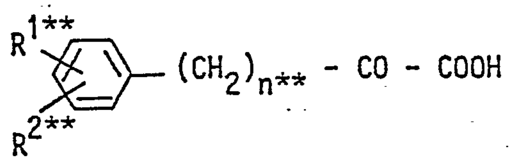 (I**),

worin $R^{1**}$ und $R^{2**}$ in meta- oder para-Stellung zum Ketocarbonsäurerest stehen und

$R^{1**}$ ein Wasserstoffatom oder ein Chloratom,

$R^{2**}$ ein Chloratom und

n** 3 bis 5 bedeuten,

und ihre pharmakologisch verträglichen Salze mit anorganischen oder organischen Basen.

5. 5-(4-Methoxyphenyl)-2-oxovaleriansäure und ihre pharmakologisch verträglichen Salze mit anorganischen oder organischen Basen zur Verwendung bei der Behandlung von Krankheiten, die auf Störungen des Glucosestoffwechsels beruhen.

6. Arzneimittel, die eine oder mehrere der substituierten Oxocarbonsäuren der allgemeinen Formel I'

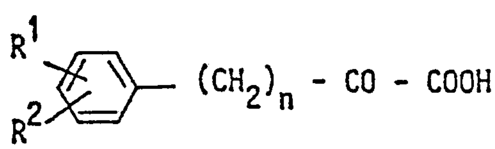 (I'),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten,

und/oder ihre pharmakologisch verträglichen Salze mit anorganischen oder organischen Basen enthalten.

7. Arzneimittel nach Anspruch 6, enthaltend substituierte Oxocarbonsäuren der allgemeinen Formel I', in der $R^1$ ein Wasserstoffatom, ein Chloratom, eine Methylgruppe, eine Methoxygruppe oder eine Trifluormethylgruppe, $R^2$ ein Wasserstoffatom oder ein Chloratom und n eine ganze Zahl von 3 bis 6 bedeuten, und/oder ihre pharmakologisch verträglichen Salze.

8. Arzneimittel nach Anspruch 6, enthaltende Verbindungen nach Anspruch 3.

9. Arzneimittel nach Anspruch 6, enthaltend Verbindungen nach Anspruch 4.

10. Verfahren zur Herstellung von substituierten Oxocarbonsäuren der allgemeinen Formel I

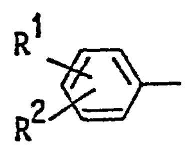 $(CH_2)_n$ - CO - COOH (I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom oder eine Methoxygruppe und $R^2$ ein Wasserstoffatom bedeuten, wenn n die Bedeutung 3 hat,

und ihren Salzen, dadurch gekennzeichnet, daß man

a) einen Oxocarbonsäureester der allgemeinen Formel II

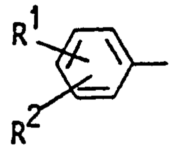 $(CH_2)_n$ - CO - CO - O - $R^3$ (II),

worin $R^1$, $R^2$ und n die vorstehend angegebene Bedeutung haben und $R^3$ einen Niederalkylrest bedeutet, solvolysiert und gegebenenfalls anschließend erhaltene Säuren in die Salze oder erhaltene Salze in die Säuren überführt, oder

b) einen Diester der allgemeinen Formel III

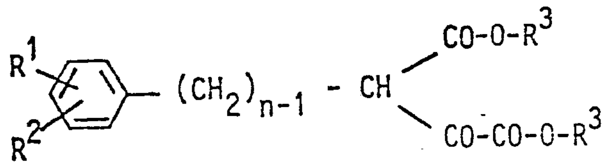

(III),

worin $R^1$, $R^2$, $R^3$ und n die vorstehend angegebene Bedeutung haben, solvolysiert und decarboxyliert und gegebenenfalls anschließend erhaltene Säuren in die Salze oder erhaltene Salze in die Säuren überführt.

11. Verfahren nach Anspruch 10, dadurch gekennzeichnet, daß man Oxocarbonsäureester II oder Diester III als Ausgangsverbindungen einsetzt, in denen n eine ganze Zahl von 3 bis 6 bedeutet, wobei nicht $R^1$ ein Wasserstoffatom oder eine Methoxygruppe und $R^2$ ein Wasserstoffatom bedeuten, wenn n die Bedeutung 3 hat.

12. Verfahren nach Anspruch 10 zur Herstellung von substituierten Oxocarbonsäuren der allgemeinen Formel I*

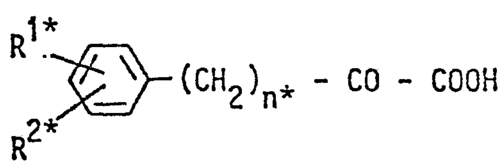

(I*),

worin

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom und

n* eine ganze Zahl von 3 bis 6 bedeuten, wobei nicht $R^{1*}$ ein Wasserstoffatom oder eine Methoxygruppe und $R^{2*}$ ein Wasserstoffatom bedeuten, wenn n* die Bedeutung 3 hat,

und ihrenSalzen, dadurch gekennzeichnet, daß man

a) einen Oxocarbonsäureester der allgemeinen Formel II*

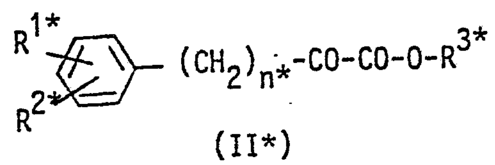

(II*)

worin $R^{1*}$, $R^{2*}$ und n* die oben angegebene Bedeutung haben und $R^{3*}$ einen Niederalkylrest bedeutet, solvolysiert und gegebenenfalls anschließend erhaltene Säuren in die Salze oder erhaltene Salze in die Säuren überführt, oder

b) einen Diester der allgemeinen Formel III*

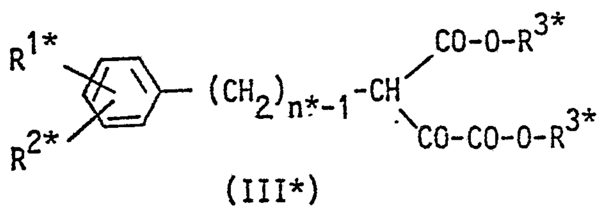

(III*)

worin $R^{1*}$, $R^{2*}$ und n* die oben angegebene Bedeutung haben und $R^{3*}$ einen Niederalkylrest bedeutet, solvolysiert und decarboxyliert und gegebenenfalls anschließend erhaltene Säuren in die Salze oder erhaltene Salze in die Säuren überführt.

13. Verfahren nach Anspruch 10 zur Herstellung von substituierten Oxocarbonsäuren der allgemeinen Formel I**

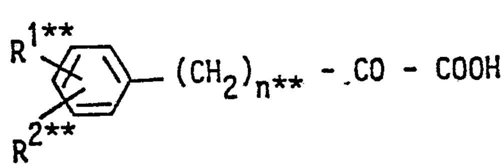

(I**),

worin $R^{1**}$ und $R^{2**}$ in meta- oder para-Stellung zum Ketocarbonsäurerest stehen und

$R^{1**}$ ein Wasserstoffatom oder ein Chloratom,

$R^{2**}$ ein Chloratom und

n** 3 bis 5 bedeuten,
und ihren Salzen, dadurch gekennzeichnet, daß man

a) einen Oxocarbonsäureester der allgemeinen Formel II**

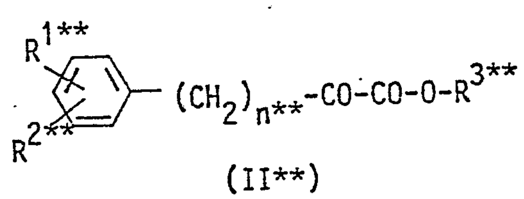

(II**)

worin $R^{1**}$, $R^{2**}$ und n** die oben angegebene Bedeutung haben und $R^{3**}$ einen Methyl- oder Ethylrest bedeutet, solvolysiert und gegebenenfalls anschließend erhaltene Säuren in die Salze oder erhaltene Salze in die Säuren überführt, oder

b) einen Diester der allgemeinen Formel III**

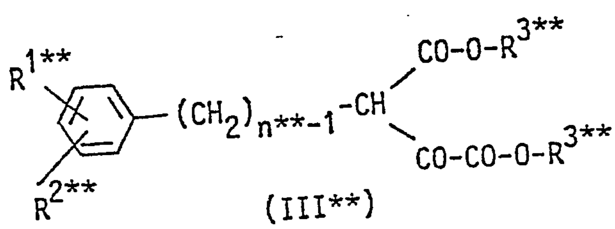

(III**)

worin $R^{1**}$, $R^{2**}$ und n** die oben angegebene Bedeutung haben und $R^{3**}$ einen Methyl- oder Ethylrest bedeutet, solvolysiert und decarboxyliert und gegebenenfalls anschließend erhaltene Säuren in die Salze oder erhaltene Salze in die Säuren überführt.

0051077

Europäisches Patentamt

# EUROPÄISCHER RECHERCHENBERICHT

Nummer der Anmeldung

EP 80 10 6676

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,A | JOURNAL OF THE AMERICAN CHEMICAL SOCIETY, Band 58, Nr. 11, November 1936, Seiten 2319-2322<br>L.F. FIESER et al.: "The synthesis of phenanthrene and hydrophenanthrene derivatives. V. The addition of dienes to cyclic α,β-unsaturated esters"<br>* Seiten 2320-2321 *<br>-- | 1,10 |
| A | DE - A - 1 810 026 (SUN OIL)<br>* Anspruch 1 *<br>---- | 1 |
| | ./. | |

KLASSIFIKATION DER ANMELDUNG (Int. Cl.³)

C 07 C 59/90
59/88
59/84
51/09
51/41
51/02
51/38
A 61 K 31/19//
C 07 C 69/738
69/65
143/68
67/343
67/333
67/317

RECHERCHIERTE SACHGEBIETE (Int. Cl.³)

C 07 C 59/90
59/88
59/84
51/09
A 61 K 31/19

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L. aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 28-01-1981 | LI VOTI |

EPA form 1503.1 06.78

0051077

Europäisches Patentamt

# EUROPÄISCHER RECHERCHENBERICHT

Nummer der Anmeldung

EP 80 10 6676

-2-

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der Maßgeblichen Teile | betrifft Anspruch | |
| | | | C 07 C 67/08<br>67/00<br>33/46<br>29/36 |
| | | | RECHERCHIERTE SACHGEBIETE (Int. Cl.3) |
| | | | |